# EUROPÄISCHE PATENTANMELDUNG

(11) **EP 0 755 921 A1**
(43) Veröffentlichungstag der Anmeldung: **29.01.1997**
(21) Anmeldenummer: 96111360.2
(22) Anmeldetag: 15.07.1996
(51) Int. Cl.: C07C 333/16, C07C 279/02, C08K 5/39, C08K 5/31

(54) **Dithiocarbamatsalze und ihre Verwendung als Vulkanisationsbeschleuniger**

(30) Priorität: 27.07.1995 DE 19527437
(71) Anmelder: BAYER AG, 51368 Leverkusen (DE)
(72) Erfinder: Lohwasser, Hermann, Dr., 41540 Dormagen (DE)

(57) **Zusammenfassung**

Salze der Formel (III) 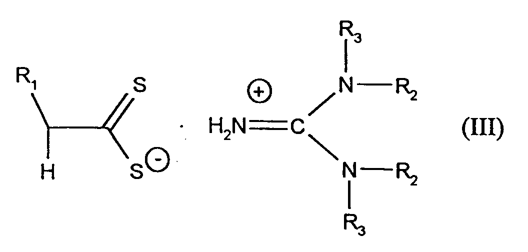 worin R₁ CN oder einen C₁-C₈-Alkylrest, R₂ einen einkernigern C₆-C₁₀-Arylrest und R₃ einen C₁-C₃-Alkylrest oder Wasserstoff bedeuten; und der Formel (IV) 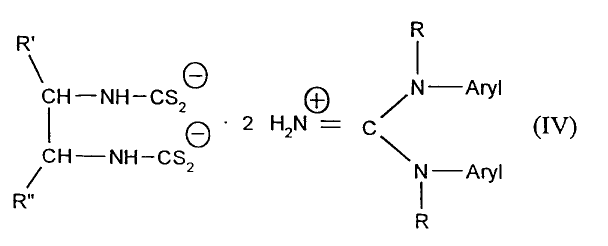 worin R Wasserstoff oder C₁-c₃-Alkyl,
Aryl einen C₆-C₁₀ (substituierten) Phenylrest,
R', R'' Wasserstoff und C₁-C₄-Alkyl bzw. R' + R'' C₄-Alkylen bedeuten und
ein Verfahren zu ihrer Herstellung durch Umsetzung des Dithiocarbamats eines primären aliphatischen Amins, eines Bis-amins oder des Cyanamids mit einem Guanidinhydrohalogenid.

## Beschreibung

Zur Vulkanisation ungesättigter Kautschuke verwendet man außer Schwefel noch Vulkanisationsbeschleuniger. Eine Gruppe solcher Beschleuniger sind die Dithiocarbamate der Formel (I) auf Basis sekundärer Amine 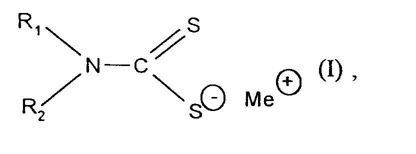 worin
- R₁ und R₂: einen C₁-C₆-Alkylrest, R₂ auch einen C₆-C₁₀-Arylrest darstellen und
- Me⁺: ein einwertiges Ammoniumkation bedeutet oder für den äquivalenten Bruchteil eines mehrwertigen Metallkations steht (z.B. 1/2 Zn⁺⁺, 1/4 Te⁺⁺⁺⁺).

Eine andere, ebenfalls geeignete Gruppe sind die stark basischen Diarylguanidine der Formel (II) 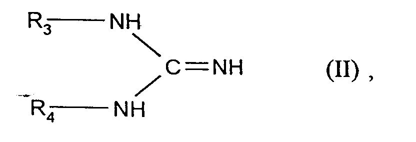 worin
- R₃ und R₄: C₆-C₁₀-Arylreste bedeuten.

Vulkanisationsbeschleuniger auf der Basis sekundärer Amine können bei der Kautschukvulkanisation durch Spaltung und Reaktion mit Stickoxiden oder Nitrit Nitrosamine bilden, denn Stickoxide befinden sich spurenweise in der Luft und auch in Bestandteilen der Kautschukmischung, beispielsweise in Form von Nitrit als Verunreinigung. Viele Nitrosamine gelten als cancerogen, so daß ihr Auftreten bei der Vulkanisation unerwünscht ist.

Ziel der Erfindung ist, Verbindungen zur Verfügung zu stellen, die als Beschleuniger für die Kautschukvulkanisation ohne die Gefahr der Nitrosaminbildung eingesetzt werden können.

Gegenstand der Erfindung sind somit Salze aus Monoalkyl- oder Alkylenbisdithiocarbamatanionen und N,N'-Diarylguanidylkationen. Diese Salze können durch die Formeln (III) und (IV) wiedergegeben werden.

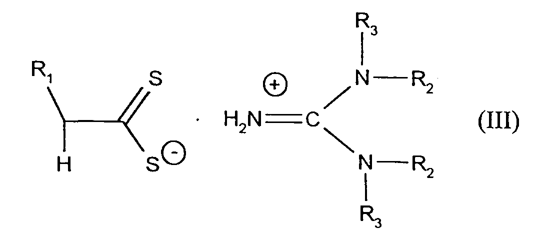

In Formel (III) bedeutet
- R₁: einen C₁-C₈-Alkylrest oder -CN (Nitrilgruppe),
- R₂: einen C₆-C₁₀-Arylrest (bevorzugt Phenyl) und
- R₃: einen C₁-C₃-Alkylrest oder Wasserstoff.

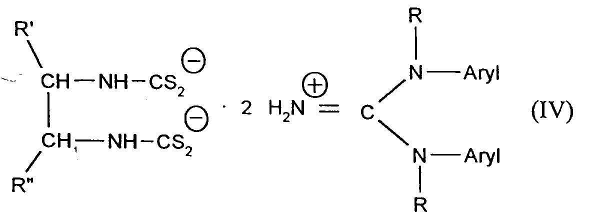

In Formel (IV) bedeutet
- R' und R'': Wasserstoff oder einen C₁-C₆-Alkylrest oder eine durch Ringschluß gebildete Alkylenkette
- R: Wasserstoff oder einen C₁-C₃-Alkylrest
- Aryl: einen C₆-C₁₀-Arylrest (vorzugsweise Phenyl).

Diese Salze können in einfacher Weise durch Umsetzung einer wäßrigen Lösung eines Ammonium- oder Alkalidithiocarbamats bzw. -bisdithiocarbamats mit einer wäßrigen Lösung eines Diarylguanidinhydrohalogenids bzw. eines Dialkyldiarylguanidinhydrohalogenids erhalten werden. Dabei fallt das Salz aus und kann in üblicher Weise abgetrennt und getrocknet werden.

Diese Umsetzung kann man bei Zimmertemperatur oder auch bei Temperaturen bis 80°C durchführen. Man wendet im allgemeinen stöchiometrische Mengen der Ausgangsprodukte an, um fast quantitative Ausbeuten zu erzielen und keines der Ausgangsprodukte am Ende der Reaktion übrigzulassen.

Beispiele für geeignete Ausgangsprodukte sind diejenigen Derivate von Alkyldithiocarbamaten bzw. Alkylen-bisdithiocarbamaten und Arylguanidin-Hydrohalogeniden, die möglichst niedrige Gesamtmolekulargewichte ergeben, insbesondere
Kalium-N-methyldithiocarbamat
Ethylen-1,2-bisdithiocarbamat-Natrium
Propylen-1,2-bisdithiocarbamat-Natrium
Butylen-2,3-bisdithiocarbamat-Ammonium
Dikalium -N-cyanodithiocarbamat
N,N'-Diphenylguanidin-Hydrochlorid
N,N'-Di-orthotolylguanidin-Hydrochlorid
N,N'-Dimethyl-N,N'-diphenylguanidin-Hydrogensulfat

Im Vulkanisationsprozeß und in ihren Vulkanisateigenschaften sind die daraus hergestellten Verbindungen den bekannten Schwermetall-dialkyldithiocarbamaten wie z.B. Zinkdiethyldithiocarbamat oder Zinkdibenzyldithiocarbamat gleichwertig und allen heute noch gebräuchlichen einfachen Dialkylammoniumdithiocarbamaten sekundärer Amine technisch überlegen. Damit stellt erst die "innere'' Kombination von Diarylguanidin mit einfach N-substituierten Dithiocarbamaten den geeigneten nitrosaminfreien Ersatz für die "klassischen" Metalldithiocarbamate dar.

### Ausführungsbeispiele

a) 1 Mol Monomethyldithiocarbamatnatrium ("Metam-Natrium") und 1 Mol Diphenylguanidinhydrochlorid (DPG-HCl) werden als wäßrige Lösungen bei 20°C miteinander umgesetzt. Es entsteht ein kristalliner Niederschlag, der einer Verbindung der Formel (IIIa) entspricht. 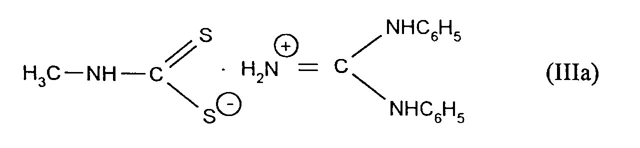
b) In gleicher Weise wie unter a) werden 1 Mol Ethylenbisdithiocarbamat-Natrium ("Nabam") und 2 Mol DPG-HCl umgesetzt. Dabei entsteht quantitativ (IVa) 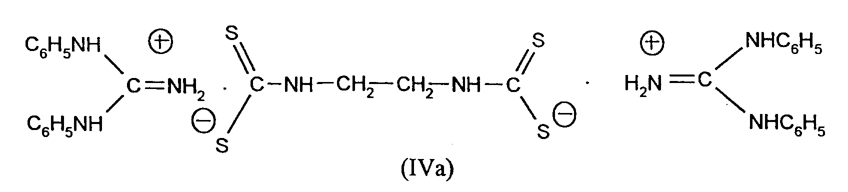
c) Bei der Reaktion bei Raumtemperatur von 1 Mol Dikalium-N-cyanodithiocarbamat mit 2 Mol Di-o-tolylguanidinhydrochlorid entsteht (IVb) als feinkristalline Fällung

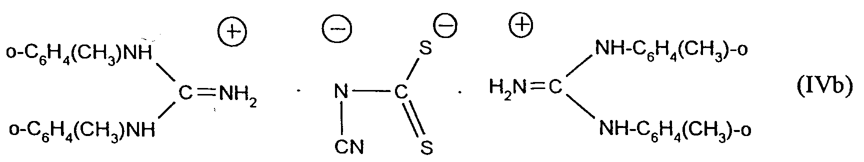

Die Substanzen (IIIa), (IVa), (IVb), als Beschleuniger in eine Gummimischung in gleicher Dosierung anstelle des beispielsweise nitrosaminbildenden Zink-di-n-butyldithiocarbamats eingebracht, liefern Vulkanisate, in denen analytisch keine "gummitypischen" N-Nitrosamine mehr nachweisbar sind.

### Referenzbeispiel

Wie im Ausführungsbeispiel (IIIa) funktioniert die Fällungsreaktion auch zwischen äquimolaren Mengen von DPG-HCl und Natrium-N.N-dimethyldithiocarbamat unter Bildung von (IIIb):

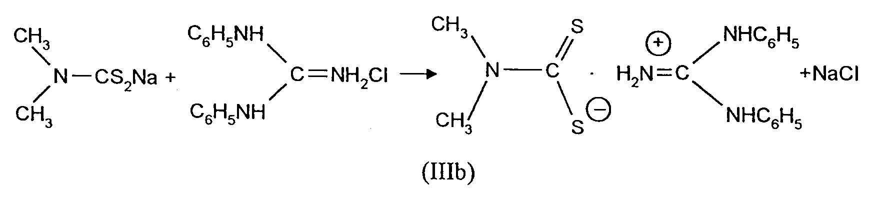

Beim Ersatz der bekannten nitrosaminbildenden Beschleunigerkombination Zinkdimethyldithiocarbamat plus Diphenylguanidin durch Substanz (IIIb) wird zwar wieder eine ausgezeichnete Beschleunigerwirkung beobachtet, aber im Gegensatz zu Verbindung (IIIa) bildet Verbindung (IIIb) während der Vulkanisation das karzinogene N-Nitrosodimethylamin, welches in der Luft von Arbeitsplätzen oder in Konsumentenartikeln grundsätzlich unerwünscht ist.

## Patentansprüche

1. Salze der Formel (III) 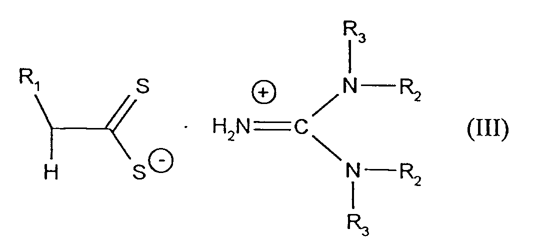 worin R₁ CN oder einen C₁-C₈-Alkylrest, R₂ einen einkernigern C₆-C₁₀-Arylrest und R₃ einen C₁-C₃-Alkylrest oder Wasserstoff bedeuten; und der Formel (IV) 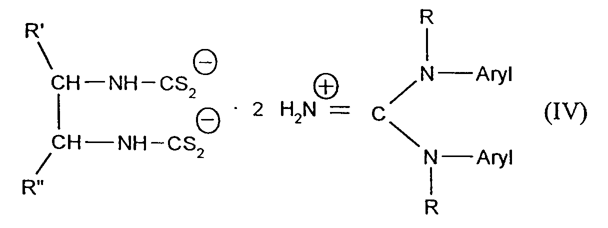 worin R Wasserstoff oder C₁-C₃-Alkyl,
Aryl einen C₆-C₁₀ (substituierten) Phenylrest,
R', R'' Wasserstoff und C₁-C₄-Alkyl bzw. R' + R'' C₄-Alkylen bedeuten.

2. Verfahren zur Herstellung der Salze gemäß Anspruch 1, dadurch gekennzeichnet, daß man das Dithiocarbamat eines primären aliphatischen Amins, eines Bis-amins oder des Cyanamids mit einem Guanidinhydrohalogenid in den erforderlichen äquimolaren Mengen in wäßriger Lösung umsetzt.

3. Verwendung der Salze gemäß Anspruch 1 als nitrosaminfreie Vulkanisationsbeschleuniger, wobei R₁ bevorzugt C₄- bis C₈-Alkyl, R₂ bevorzugt Phenyl, R₃ bevorzugt Wasserstoff bzw. R' bevorzugt Methyl oder Wasserstoff, R'' und R bevorzugt Wasserstoff, Aryl bevorzugt Phenyl oder ortho-Tolyl darstellen.
